# EUROPEAN PATENT APPLICATION

(11) **EP 2 581 080 A1**
(43) Date of publication of application: **17.04.2013**
(21) Application number: 11185029.3
(22) Date of filing: 13.10.2011
(51) Int. Cl.: A61K 31/194, A61P 31/04, A61Q 17/00

(54) **Inhibitor of colonisation of mucosa**

(71) Applicant: Helmholtz-Zentrum für Infektionsforschung GmbH, 38124 Braunschweig (DE)
(72) Inventor: Steindorff, Marina, 38118 Braunschweig (DE); Tegge, Werner, 38126 Braunschweig (DE)
(74) Representative: Kröncke, Rolf

(57) **Abstract**

The present invention relates in the first aspect to the use of aurintricarboxylic acids, derivatives, oligo- or polymers or salts, for inhibiting adherence and colonisation of microorganisms, like bacteria, on surfaces, in particular for use in mammals. That is, it has been recognized that aurintricarboxylic acids and derivatives thereof are suitable for inhibiting adherence and colonisation of mucosa, in particular, nasal mucosa. In another aspect, the present invention relates to a method for preventing adherence of microorganisms, like bacteria, to a surface comprising the step of treating the surface with aurintricarboxylic acids, derivatives, or salts thereof. Finally, the present invention relates the use of aurintricarboxylic acids, derivatives or salts for preventing adherence of bacterial pathogens, in particular, of the genus *Staphylococcus* on surfaces.

## Description

The present invention relates in the first aspect to the use of aurintricarboxylic acids, derivatives, oligo- or polymers or salts thereof, for inhibiting adherence and colonisation of microorganisms, like bacteria, on surfaces, in particular for use in mammals. That is, it has been recognized that aurintricarboxylic acids and derivatives thereof are suitable for inhibiting adherence and colonisation of mucosa, in particular, nasal mucosa. In another aspect, the present invention relates to a method for preventing adherence of microorganisms, like bacteria, to a surface comprising the step of treating the surface with aurintricarboxylic acids, derivatives, or salts thereof. Finally, the present invention relates the use of aurintricarboxylic acids, derivatives or salts for preventing adherence of bacterial pathogens, in particular, of the genus *Staphylococcus* on surfaces, in particular, human body surfaces.

### Prior Art

Adhesion of microbial represents one of the initial steps for colonisation and subsequent infection with said microorganism, in particular, microbial pathogens.

After initial adhesion of the microbial pathogens, the microorganisms try to subvert and subjugate the host cells for their pathogenesis. That is, adhesion refers to the attachment of the microorganisms, in particular, bacteria on surfaces including human body surfaces, like mucosa, representing one of the first initial steps of the overall infection process. As used herein, the term mucosa refers to all linings involved in absorption and secretion. They line cavities that are exposed to the external environment and internal organs. Typically, they produce and secrets mucus. The mucosae include mucosal tissue or mucosal membranes in the nostril, the mouth, the eyelids, the ears, the genital area, the anus and throat. In addition, the lining separating inner organs from the environment, e. g. in the intestine etc. is mucosa. In contrast to skin the mucosa does not have an epidermis but is built mainly by epithelial cells.

For example, the gram-positive genus of *Staphylococcus* like *Staphylococcus aureus,* a facultative anaerobic coccal bacterium, is frequently part of the endogenous microflora found on mammalian body surfaces, like human body surfaces, e.g. on mucosa and on skin. About 20 % of the human population is a constant carrier of *S. aureus. S. aureus* is the most common species of *staphylococcae* to cause *Staphylococcus* infection. Typically, the nasal vestibule or nasal cavity is the preferred area of *S. aureus* persistence. About 60 % of the human population is a sporadic carrier while about 20 % is never a carrier of *S*. *aureus.* In addition, *S. aureus* may occur in the pharynx or in the armpits. While healthy persons populated with *S. aureus* do not show any symptoms and even do not know that there are carrier for said bacteria, people who have a depressed or suppressed immune system, like HIV-patients or people obtaining immunosuppressants but also elder people and people having a chronic disease are at risk of microbial infection. *S. aureus* can cause a range of illnesses from minor infections of skin and mucosae. For example, infection by *S. aureus* may be the cause for local superficial infection of the skin, like inflammations or ignitions, but may also cause gastroenteritis, infection of the urinary tract and infection of the upper airways. But also more severe and life-threatening diseases may occur, such as pneumonia, meningitis, osteomyelitis, endocarditis, toxic shock syndrome, bacteraemia and sepsis. Its incidence is from skin, soft tissue, respiratory, bone, joint, endovascular to wound infections. *S. aureus* is still one of the five most common causes of nosocomial infections, often causing postsurgical wound infections. For example, some 500.000 patients in American hospitals show a staphylococcal infection each year. Today methicillin-resistant *S. aureus* (MRSA strains), one of the well-know multi-drug resistant *S. aureus* strains (MDRSA strains), are one of a number of greatly-feared strains of S. *aureus,* which have become resistant to most antibiotics. MRSA strains are most often found associated with institutions such as hospitals, but are becoming increasingly prevalent in community-acquired infections. For example, S. *aureus* present in meat and poultry may be responsible for severe infections of humans by S. *aureus.* In this connection, it was recognized that S. *aureus* contaminating meat and poultry are becoming more and more resistant to antibiotics, e. g. representing MRSA strains.

S. *aureus* infections may spread through contact with pus from an infected wound, skin-to-skin contact with an infected person and contact with objects such as towels, sheets, clothing's or other equipment used by an infected person. That is, spread of *S. aureus* including MRSA is generally through human contact. In addition, *S. aureus* infection, in particular MRSA infection represents a major threat in hospitals. Often, spread of *S. aureus* may be facilitated by insufficient healthcare worker hygiene. Further, spreading of *S. aureus* may occur through medical instruments and medical devices.

Thus, there is a need to control spreading and colonisation of *S. aureus,* in particular, in hospitals and other institutions where people having a suppressed immune system are staying.

In addition a major goal is an eradication of *S. aureus* in hospitals and other healthcare institutions to avoid any life-threatening infections of patients and persons having a suppressed immune system or elder persons. Today the active mupirocin represents the treatment of choice for eradication of *S. aureus.* Typically, mupirocin is administered topically as an ointment or salve. The problem to control infection with *S. aureus* is that *S. aureus* has become resistant to many conventionally used antibiotics. Hence, for treating MRSA infection the use of new antibiotics or new strategies is required. A preventive measure to prevent *S. aureus* infection is to eradicate the nasal colonisation of *S. aureus,* in particular of the nasal vestibule. It has been demonstrated that eradication of *S. aureus* from the nasal vestibule leads to a general reduction of the colonization of other areas of the body. Mupirocin as the treatment of choice allows eradicating of S. *aureus* from the nasal mucosa or other mucosa. However, administration of mupirocin is contraindicated for pregnant women and children. Moreover, mupirocin resistant strains are emerging and have been isolated already, thus, requiring the development of new components allowing eradication of S. *aureus* or other microbial pathogens.

Lee, J.-H., et al., Carbohydrate Research, 2006, 341, 1154-1163 describes a pectin-like acidic polysaccharide from *Panex ginseng* having a selective antiadhesive activity against pathogenic bacteria. Further, Weidenmaier, C., et al., Int. J. Med Microbiol, 2008, 298, 505-513, discloses an antiadhesive activity of polyinosinic acid (polyl) on binding of S. aureus on primary human nasal epithelial cells.

Aurintricarboxylic acid (ATA) is a substance formed when salicylic acid is treated with sulfuric acid, formaldehyde, and sodium nitrite. ATA is known as a potent inhibitor of many biochemical processes that are dependent upon the binding of nucleic acids and proteins. For example, it is demonstrated in the art, that ATA inhibits a variety of enzymes that process nucleic acids. In addition, ATA inhibits protein synthesis by interfering with a binding of mRNA to ribosomes and it also inhibits the binding of glucocorticoid-receptor complexes to DNA. Further, it is described that ATA has an anticryptosporidial activity, Kleine, P. et al, J. antimicrobial chemotherapy, 2008, 62, 1101 - 1104. In addition, ATA is described as having inhibiting activity on angiogenesis and on blood coagulation as well as having an activity to strongly inhibit the replication of virus, like SARS.

In WO 2005/123965 the potent and selective inhibition of ATA on SARS is described. WO 2008/151412 describes a synergistic inhibition of H1N1 and H3N2 influenza viruses by ATA with amantadine. WO 94/26278 relates to the use of ATA and analogues of having anti-angiogenic activity and its potential usefulness for the treatment of diseases being dependent on angiogenesis, arthritis, tumorgenesis and metastasis as well as neovascular glaucoma.

Recently, Hashem, A. M., et al, Plos. 1, 2009, 4, 12, 1 - 10 describe that ATA is a potent inhibitor of influenza A and B virus neuraminidases.

Hence, there is still an ongoing need for compounds and compositions allowing to prevent or treat microbial infection and to provide new compounds allowing eradication of microbial colonisation e. g. of microbial populations of *S. aureus.*

### Brief description of the present invention

In a first aspect, the present invention relates to a composition for preventing or inhibiting adherence of microorganisms, in particular, bacteria, on surfaces, like body surfaces including skin or mucosa of mammals, like human body surfaces.

In particular, the present invention relates to a pharmaceutical composition containing aurintricarboxylic acid, derivatives, or salts thereof for use in inhibiting or preventing adherence of microorganisms for preventing or treating microbial infections, in particular, bacterial infection, like infection by the genus *Staphylococcus.* In particular, the present invention is useful for preventing or inhibiting adherence and, consequently, potential infection with *Staphylococcus aureus,* in particular, MRSA.

The composition is particularly useful for nasal administration, in particular, for nasal application for use in preventing or treating colonisation of the nasal mucosa by bacterial pathogens, in particular, of *Staphylococcus aureus.*

In another aspect, a method for preventing adhesion of microorganisms to or on a surface, in particular, for preventing adherence of *S. aureus* to or on surfaces of medical devices and medical instruments comprising the step of treating the surface with aurintricarboxylic acids, derivatives, or salts, or polymers of aurintricarboxylic acid is provided.

### Brief description of the drawings

Figure 1 shows the results on the inhibiting activity of ATA (a), pseudohypericine (b) and polyl (c) on the adherence of S. *aureus* N315 on A549 cells. The vertical lines relate to the IC50 and IC90 values, respectively, of the tested compounds.
Figure 2 demonstrates that ATA is able to reduce adherence of S. *aureus* on A549 cells. As shown in figure 2, the percentage of adherence of S. *aureus* can be reduced to below 40% compared to the control with no ATA after 3 hours at concentrations as low as 2.2 µg/ml. Black bars represent 0.95 µg/ml ATA and white bars represent 2.2 µg/ml. A549 cells were incubated with S. *aureus* for 1 hour in advance and then ATA was added. Shown are the results for adherence after incubation with ATA for the time period indicated in the graph.
Figure 3 shows the inhibiting activity on different strains of *S. aureus* of ATA and pseudohypericine using different concentrations of ATA and pseudohypericine.
Figure 4 demontrates that ATA is able to inhibit adhesion of *S. aureus* N315 on human primary nasal epithelial cells. The vertical line shows the IC50 value of ATA.

### Detailed description of the present invention

In a first aspect, the present invention relates to aurintricarboxylic acid, derivatives or salts thereof for use in preventing or inhibiting adherence of microorganisms on surfaces. In particular, the present invention relates to the usefulness of aurintricarboxylic acid (ATA), derivatives or salts for use in preventing or inhibiting adherence of bacteria on surfaces.

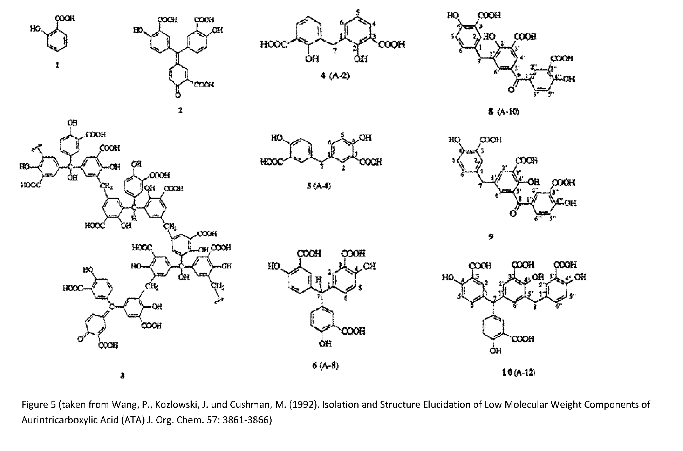

As used herein, the term aurintricarboxylic acid, in the following also abbreviated with the term "ATA", includes derivatives or salts thereof unless otherwise indicated. In addition, ATA includes not only the monomeric aurintricarboxylic acid but also oligomeric and polymeric forms thereof, including rearrangement products. Examples of the various forms of ATA are shown in figure 5 derived from Wang, P., et al., J. Org. Chem. 1992, 57, 3861-3866 which is included by reference herewith. The basic structure of ATA is shown as formula I

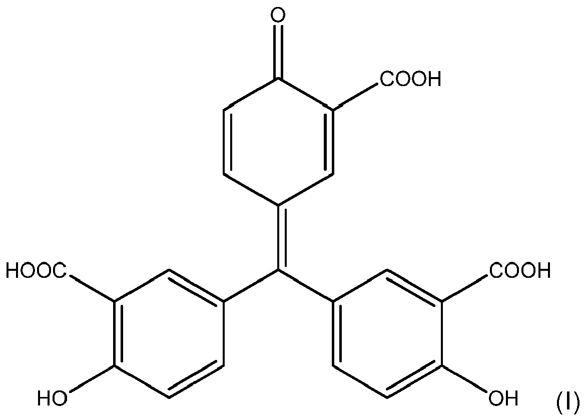

As used herein, the term "aurintricarboxylic acid derivatives" or "ATA derivatives" includes chemically modified variants thereof. That is, compounds having the same general structure as ATA, its oligo- and polymers and its rearrangement products and further having the same biological activity as ATA and pharmaceutically acceptable salts thereof, wherein "biological activity" refers to the inhibition of adherence of microorganisms.

As used herein "inhibition" in all its grammatical forms does not imply a complete cessation but rather indicates that the adherence being inhibited occurs at a lower rate or efficiency.

In addition, as used herein, "prevention" in all its grammatical forms does not imply a complete prevention but rather indicates that the adherence being prevented occurs at a lower rate or efficiency.

As used herein, the term "adherence" refers to attachment of microorganisms, in particular of bacteria, on surfaces, e.g. human body surfaces, like mucosae. In infection processes, adherence is one of the major initial steps allowing the pathogen to invade the host.

In a preferred embodiment of the present invention, ATA is useful in preventing or inhibiting adherence of microorganisms, like bacteria, on surfaces preferably body surfaces of mammals, like human body surfaces. That is, ATA allows preventing or inhibiting adherence of microbial pathogens on body surfaces, like skin or mucosae. It is preferred that the microorganism is a bacterium, in particular, a bacterial pathogen. In a particular preferred embodiment, the present invention relates to a pharmaceutical composition containing ATA for use in inhibiting or preventing adherence in order to prevent or treat microbial infections, in particular, bacterial infections by the genus *Staphylococcus.*

In a particular preferred embodiment, the bacterium which adherence should be inhibited or prevented is a multi-drug resistant bacterium, like a methicillin-resistant bacterium, in particular a multi-drug resistant *Staphylococcus aureus* strain (MDRSA) like e.g. MRSA. That is, ATA is particularly useful for preventing or treating colonisation by MRSA. MRSA is one of a member of greatly feared strains of *S. aureus* which have a common resistance to most antibiotics. Further, MRSA strains are most often found associated with institutions such as hospitals, but are becoming increasingly prevalent in community acquired infections. As used herein, the term "MDRSA" refers to multi-resistant *Staphylococcus aureus* strains not only being resistant to methicillin (MRSA, methicillin-resistant *S. aureus*) but may be in addition or alternatively resistant to other antibiotic classes. In Germany about 160,000 patients in hospitals are infected with MRSA. MRSA infection may be caused due to low hygiene in hospitals and low standards of hygiene. Hence, it is important to provide new means for preventing spreading and circulation of MRSA in hospitals and to eradicate MRSA in hospitals and other places where MRSA spreading may occur.

Hence, the composition is preferably in form of an ointment, tincture, salve, spray, aerosol, gel, emulsion, solution or suspension. A preferred way of administration includes topical or mucosal application. In a preferred embodiment, the composition according to the present invention is adapted for nasal administration, in particular, in form of a nasal salve, nasal spray or nasal ointment.

In a particular preferred embodiment, the present invention relates to a composition for use in preventing or treating colonisation of nasal mucosa, in particular, in the nasal vestibule or nasal cavity by bacterial pathogens of the genus *Staphylococcus,* in particular, of *Staphylococcus aureus,* most preferably, of MRSA. The composition containing ATA is provided in form of a nasal salve, nasal spray or nasal ointment to be administered to the nasal vestibule in order to prevent adherence or inhibit adherence of the bacteria. In particular, the composition is useful for eradicating colonisation by *Staphylococcus,* like *Staphylococcus aureus.* The composition in form of the nasal salve, nasal spray or nasal ointment is of particular usefulness in hospitals and nursing homes for the elderly for treating patients and to increase the hygiene standards.

It is preferred that the ATA is aurintricarboxylic acid having a molecular weight of about 422 daltons and/or oligomers, polymers and rearrangement products of the former.

In another embodiment, the present invention relates to a method for preventing adhesion of microorganisms, in particular, of bacteria on a surface of a device or on a surface of a mammalian body surface, e.g. human body surface, or, alternatively, except of human body surfaces. Said method comprises the step of treating the surface with ATA. It has been demonstrated herein that ATA allows to decrease adherence of microorganisms, in particular, of *Staphylococcus,* like *Staphylococcus aureus* on surfaces. It is preferred that the method is used for preventing or inhibiting adherence of bacterial pathogens, like *Staphylococcus aureus,* in particular, of MRSA strains.

Preferably, the surface is the surface of catheters and medical instruments, like surgical instruments, or medical implants, medical prothesis, medical devices or medical auxiliaries. Implementing treatment of surfaces of medical instructions, medical implants, medical prothesis, medical device or medical auxiliaries may allow to prevent or treat colonisation and spreading of bacterial pathogens, like of the genus *Staphylococcus,* in particular, of *Staphylococcus aureus.* Further, the method may allow to eradicate S. *aureus* in hospitals. Finally, the present invention relates to the use of ATA for preventing adherence of microorganisms, like bacterial pathogens, in particular, of the genus *Staphylococcus* on surfaces, in particular, on mucosa or skin of mammals.

Of course, the pharmaceutical composition may optionally comprise pharmaceutically acceptable carrier, diluents and/or recipients.

The pharmaceutical composition may be administered with a physiologically acceptable carrier to a patient, as described herein. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds, or salts thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

The term "administered" means administration of a therapeutically effective dose of the aforementioned pharmaceutical composition as defined herein to an individual. By "therapeutically effective amount" is meant a dose that produces the effects for which it is administered. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

In addition, the pharmaceutical composition described herein may be characterized in that the components of the pharmaceutical composition are associated and/or incorporated and/or coated to a physical particle, preferably microparticle, nanoparticle, liposome, ISCOM, copolymer and/or biological particle.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

In vitro assays may optionally be employed to help identifying optimal dosage ranges. The precise dose to be employed in the formulation will also depend on the route of administration, and the seriousness of the disease or disorder, and should be decided according to the judgement of the practitioner and each patient's circumstances. Effective doses may be extrapolated from dose-response curves derived from in vitro or animal model test systems. Preferably, the pharmaceutical composition is administered directly or in combination with an adjuvant. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

The compositions, like the pharmaceutical compositions as well as the methods are applicable to both human therapy and veterinary applications. The compounds described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt%. The agents may be administered alone or in combination with other treatments.

The attending physician and clinical factors will determine the dosage regimen. A typical dose can be, for example, in the range of 0.001 to 1000 µg; however, doses below or above this exemplary range are envisioned, especially considering the aforementioned factors.

That is, the present invention relates further to a method of preventing or treating microbial colonisation of body surfaces of mammals, like humans, including the step of administering therapeutically effective amounts of ATA to said mammal.

In addition, the term "containing" or "comprising" includes embodiments where the pharmaceutical composition consist of the respective components are identified.

The present invention will be described further by examples without limiting the same thereto.

### Examples

### General:

### Bacterial culture

*S. aureus* (N315) was precultivated on blood agar plates at 37°C overnight. A colony was picked from the plate with a sterile loop and transferred into 5 ml of BHI medium. The suspension was incubated aerobically at 37°C for 18 h. After this time the bacterial suspension was centrifugated and the clear medium was decanted off. The pellet was resuspended in PBS and centrifugated again twice. Finally the pellet was resuspended in infection medium at an OD of 1.0 at 600 nm.

Infection medium: Gibco RPMI 1640medium, pH 7.4 (Invitrogen, Carlsbad, USA), containing 1 % FBS and 20 mM Hepes.

### Cell culture and assay

All media that were used were prewarmed to 37°C. The cell line A-549 was utilized. A-549 cells from a stock that was stored at -196°C were quickly thawed and suspended in 10 ml of cell culture medium (DMEM (Lonza, Basel, Schweiz) containing 10 % FBS (Invitrogen, Carlsbad, USA)). They were cultivated at 37°C with 10% CO2 in cell culture bottles with a surface of 25 cm² under moisture saturation. The next day, the medium was removed and fresh medium was added. The cells were harvested at a confluency state of 80-90% by scrapping them off the bottom of the bottles and pipetting the suspension up and down several times. An aliquot was transferred into a fresh cell culture bottle with fresh medium. After another round of growth to 80-90% confluency the medium was removed and the cell layer was washed with 10 ml of sterile EBSS (Invitrogen). After removing the liquid, 1 ml of a 1% solution of trypsin was evenly distributed over the cell layer. After an incubation for 5 min at 37°C and a visible detachment of the cell layer, 10 ml of fresh cell culture medium were added, the cells were resuspended and counted in a Neubauer cell counting chamber. A cell density of 100,000 / ml was adjusted and 100 µl of the cell suspension was distributed into the wells of a 96-well microtiter plate. The plate was incubated at 37°C with 10% CO2 and moisture saturation until the cells had formed an adherent monolayer with 95-100% confluency (4-5 days). The medium was removed by pipetting and 75 µl of fresh infection medium were added to each well, followed by 25 µl of a bacterial suspension (OD600 1.0), together with different concentrations of the test compound. After an incubation time of 60 min at room temperature the medium and non-adherent bacteria were removed with a pipette and the wells were washed three times with PBS.

For fixing the cells a solution of 4% paraformaldehyde in PBS was added and incubated for 20 min at room temperature. After removing the solution and washing the cells with PBS twice, a primary antibody against *S*. *aureus* lipoteichoic acid (from rabbit, Acris Antibodies GmbH, Herford) at 1:5000 dilution in 1% BSA-PBS was added (50 µl/Well) and incubated for 45 min at room temperature. During the last 10 min of this incubation an additional 50 µl/well of a solution of DAPI ((4',6 Diamidino-2-phenylindol) dihydrochloride) in PBS (final concentration 1 µg/ml) was added. After a washing step an incubation with the HRP-conjugated secondary antibody (goat anti rabbit, Dianova) in 1% BSA-PBS for 45 min at room temperature was carried out. For detection of chemiluminescence the BM chemiluminescence ELISA substrate (POD - peroxidase-based secondary detection systems, Roche) was used. Chemiluminescence was determined immediately after addition of the substrate with a chemiluminescence microtiter plate reader.

The detection and quantification of the adherent bacteria can also be carried out with a fluorescence-coupled secondary antibody, for which Alexa Fluor 488 goat anti rabbit at 1:1000 dilution was used. The determination of fluorescence was done after washing with PBS and adding 100 µl PBS per well with a fluorescence microtiter plate reader at 485/530 nm.

Another and very favourable way to quantify the adherent bacteria is the utilization of an automatic microscope.
The procedure is described for the ImageXpress Micro (IXM, Molecular Devices)
Objectice 20-fold; binning 2; camera gain 1. The laser-based autofocus of the instrument was employed. Nine positions per well were investigated which were positioned in the middle area of the well bottoms; from those nine sites the average bacterial count per eucaroytic cell was determined. For the automatic detection and quantification of the eucaryotic cells and the bacteria the modul "Transfluor" of the instrument software MetaXpress was used.

The experiments with the human nasal primary cells (HNEPC, Provitro GmbH, Berlin) were used as described for the A-549 cells and used for up to four passages (corresponding to approx. four weeks). For the HNEPC, Airway epithelial cell growth medium (Provitro GmbH, Berlin) was used.

### Example 1

Identification of compounds having inhibitory activity on adherence of *S*. *aureus.*

Following the general approach described above, aurintricarboxylic acid and pseudohypericine have been identified as candidate compounds having an inhibitory activity on adherence of *S*. *aureus* after screening of various libraries.

**Table 1:**

| **substance** | **Reduction of adhesion [%]** | |
|---|---|---|
| | **100 µM** | **≤ 50 µM** |
| aurintricarboxylic acid (ATA)* | > 90 | > 90 |
| pseudohypericine | > 90 | 50 |

**Table 2: IC-values of candidate compounds**

| **substance** | **IC₁₀** | **IC₅₀** | **IC₉₀** |
|---|---|---|---|
| aurintricarboxylic acid | ∼0,45 µg/ml | 0,95 µg/ml | ∼2,2 µg/ml |
| pseudohypericine | 20 µM | 51 µM | 80 µM |

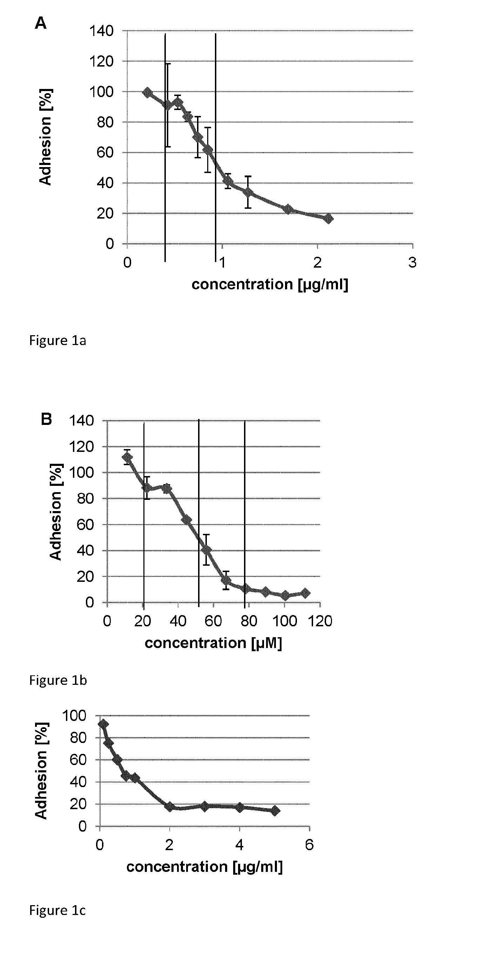

The data on the adhesion of *S. aureus* N315 as determined with the adhesion test described above in the presence of different concentrations of the candidate compounds is shown in figure 1 a and 1b. Figure 1c shows a comparative example using polyinosinic acid as described in Weidenmaier, C., et al., Int. J. Med Microbiol, 2008, 298, 505-513.

### Example 3

### Influence of ATA on adhering S. aureus

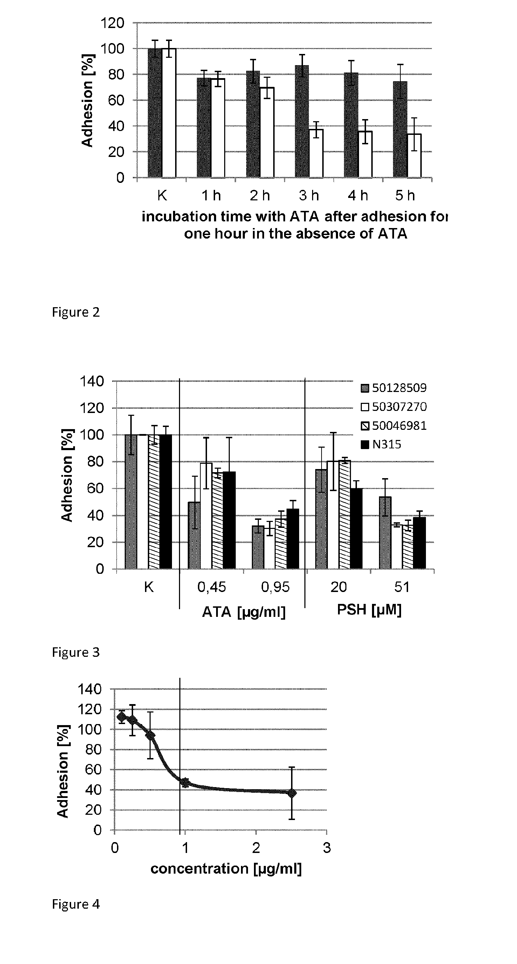

Next, the influence of ATA at two different concentrations corresponding to the IC50 and IC90 values determined in Example 2, on adhering *S*. *aureus* cells has been determined. That is, the *S*. *aureus* cells were allowed to adhere to the A549 cells for 1 hour and, thereafter, ATA was added in the respective concentrations. Figure 2 shows the results after incubation with ATA for a period of time as identified in figure 2. The adhesion has been calculated on the basis of 100% adhesion in the absence of ATA. It is clear from the results that ATA is a potent inhibitor of adherence and, in addition, is potent in eradicating *S*. *aureus* after adherence to cells.

### Example 4

### Comparison of inhibiting properties of different S. aureus isolates

The inhibitory properties of ATA on different *S. aureus* isolates have been determined using the following strains:50128509 (MRSA; source: HZI Braunschweig), BH30(04) (MRSA; biofilm positive; SCCmec type II; MLST type 8; CC8; kindly provided by Prof. Jim O'Gara), 50307270 (rifampicin-resistant; source HZI Braunschweig), BH30(04) spa (tetracycline resistant; kindly provided by Prof. Jim O'Gara), 50046981 (methicllin sensitive; source: HZI Braunschweig).

Adherence has been determined as described above. Figure 3 shows the results obtained. The results demonstrate that ATA is effective on different types of *S*. *aureus* strains.

### Example 5

### Inhibition of adherence of S. aureus on primary nasal epithelial cells

Following the general procedure as described above, the properties of ATA on inhibiting adhesion of *S*. *aureus* N315 on human primary epithelial cells was investigated. ATA was added at different concentrations to the culture and adherence was determined as described before. The results are shown in figure 4. Already a concentration of 1 µg/ml ATA is sufficient to reduce the adherence of *S*. *aureus* to about 45%.

As demonstrated herein, ATA is a component that inhibits or decreases adherence of *S. aureus,* like the MRSA strain *S. aureus* N315, on epithelial cells. Thus, ATA is a compound suitable for use in inhibiting or preventing or reducing adherence of microorganisms, like *S. aureus.* ATA should be of particular help in preventing and treating including persistence to microbial infection, like infection by *S. aureus,* in particular, MRSA strains. Further, ATA should be useful in preventing or cleaning medical devices, like catheters, or other medical auxiliaries.

## Claims

1. Aurintricarboxylic acid, derivatives, or salts thereof for use in preventing or inhibiting adherence of microorganisms, like bacteria, on surfaces, in particular for use on mammalian body surfaces, like human body surfaces.

2. Pharmaceutical composition containing aurintricarboxylic acid, derivatives, or salts, for use in inhibiting or preventing adherence for preventing or treating microbial infections, in particular, bacterial infections by the genus *Staph ylococcus.*

3. The composition according to claim 1 or 2, wherein the bacteria are multi drug resistant bacteria.

4. The composition according to any one of the preceding claims, wherein the bacterium is a multi-drug-resistant *Staphylococcus aureus,* like MRSA.

5. A composition according to any one of the preceding claims for topical or mucosal application.

6. A composition according to any one of the preceding claims, wherein the composition is administered in the form of an ointment, salve, tincture, aerosol, spray, gel, emulsion, solution or suspension.

7. A composition according to any one of the preceding claims, wherein the composition is adapted for nasal administration, in particular, in form of a nasal salve, nasal spray or nasal ointment.

8. A composition according to any one of the preceding claims for use in preventing or treating colonisation of nasal mucosa by bacterial pathogens of the genus *Staphylococcus,* in particular, of *Staphylococcus aureus,* preferably, of MRSA.

9. The composition according to any one of the preceding claims, wherein the aurintricarboxylic acid is an aurintricarboxylic acid having a molecular weight of about 422 dalton and/or oligomers or polymers thereof or rearrangement products of the former.

10. The composition according to any one of the preceding claims for use in the eradication of *S*. *aureus.*

11. A method for preventing adhesion of microorganisms, in particular, bacteria on a surface comprising the step of treating the surface with aurintricarboxylic acid, derivatives or salts thereof.

12. The method according to claim 11 for inhibiting adherence of bacterial pathogens, in particular, of the genus *Staphylococcus,* like *Staphylococcus aureus,* in particular, of MRSA.

13. The method according to claim 11 or 12, whereby the surface is the surface of a catheter or medical instrument, in particular, surgical instrument, implant, prothesis, medical device, like catheters, or medical auxiliary.

14. The use of aurintricarboxylic acid, derivatives or salts for preventing adherence of microorganism, like bacterial pathogens, in particular, of the genus *Staphylococcus* on surfaces, in particular, human body surfaces.
